# EUROPÄISCHE PATENTSCHRIFT

(11) **EP 2 482 852 B1**
(45) Veröffentlichungstag und Bekanntmachung des Hinweises auf die Patenterteilung: **22.11.2017**
(21) Anmeldenummer: 10760673.3
(22) Anmeldetag: 29.09.2010
(51) Int. Cl.: A61K 47/61, A61K 9/00, A61K 9/06, A61K 47/32, A61K 47/34, A61K 47/36, A61K 47/38, A61K 9/20, A61K 8/73, A61Q 19/00, A61K 8/81, A61K 47/58

(54) **MUKOADHÄSIVE POLYMERE MIT VITAMIN B-TEILSTRUKTUREN**
MUCOADHESIVE POLYMERS HAVING VITAMIN B PARTIAL STRUCTURES
POLYMÈRES MUCO-ADHÉSIFS COMPRENANT DES STRUCTURES PARTIELLES DE VITAMINE B

(30) Priorität: 30.09.2009 AT 15402009
(43) Veröffentlichungstag der Anmeldung: 08.08.2012
(73) Patentinhaber: ThioMatrix Forschungs- und Beratungs GmbH, 6020 Innsbruck (AT); Croma Pharma GmbH, 2100 Leobendorf (AT)
(72) Erfinder: LEIERER, Johannes, A-6020 Innsbruck (AT)
(74) Vertreter: Schwarz & Partner Patentanwälte OG
(86) Internationale Anmeldenummer: PCT/EP2010/064464
(87) Internationale Veröffentlichungsnummer: WO 2011/039259

(56) Entgegenhaltungen:
- EP-A1- 0 305 749
- WO-A1-00/25823
- WO-A1-01/32623
- WO-A1-96/07676
- CS-B1- 200 754
- US-A- 5 763 579
- LEITNER V M ET AL: "Thiolated polymers: evidence for the formation of disulphide bonds with mucus glycoproteins", EUROPEAN JOURNAL OF PHARMACEUTICS AND BIOPHARMACEUTICS, ELSEVIER SCIENCE PUBLISHERS B.V., AMSTERDAM, NL, Bd. 56, Nr. 2, 1. September 2003 (2003-09-01), Seiten 207-214, XP004453354, ISSN: 0939-6411, DOI: 10.1016/S0939-6411(03)00061-4

## Beschreibung

Mukoadhäsive Polymere sind polymere Verbindungen, die sich durch ein vergleichsweise hohes Haftvermögen an Schleimhäuten auszeichnen. Zu mukoadhäsiven Polymeren zählen Verbindungen wie (quervernetzte) Poly(meth)acrylate, (trimethylierte) Chitosane, Hyaluronsäure, Alginate, Pektine und Zellulosederivate. Die Quervernetzung von Poly(meth)acrylaten erfolgt während der Polymerisation durch Zugabe von Verbindungen, die zumindest zwei Vinylteilstrukturen wie z.B. Divinylglykol oder Pentaerythritolallylether aufweisen. Zellulosederivate mit mukoadhäsiven Eigenschaften sind vor allem Natrium Carboxymethylzellulose, Hydroxyethylzellulose, Ethylzellulose, Methylzellulose und Hydroxypropylmethylzellulose. Durch die verbesserte Anhaftung von Wirkstoffabgabesystemen an Schleimhäuten wie z.B. der des Dünndarmes, über die die Wirkstoffaufnahme erfolgt, können Wirkstoffe vergleichsweise effizienter und langanhaltender in den Blutkreislauf eingeschleust werden. Dieser große Vorteil von mukoadhäsiven Polymeren gegenüber derzeit verwendeten Wirkstoffabgabesystemen konnte bereits in verschiedenen Studien eindeutig bewiesen werden [z.B. Akiyama et al., J. Pharm. Pharmacol., 50 (1998) 159-166].

Durch die kovalente Bindung von Thiolteilstrukturen an solche Polymere können deren mukoadhäsiven Eigenschaften noch zusätzlich deutlich verbessert werden, da diese Thiolgruppen mit Thiolteilstrukturen im Mukus kovalente Bindungen in Form von Disulfidbrücken ausbilden können [Muco-adhesive polymers, use thereof and method for producing the same. EP 1126881].

Polymere mit Thiolteilstrukturen - kurz *Thiomere* (thiolated polymers) genannt - zeigen ein um mehr als das 100-fache verbesserte Anhaftungsvermögen an verschiedenen Schleimhäuten [z.B. Roldo et al., Eur. J. Pharm. Biopharm., 57, 115-121].

Trotz Thiolisierung sind die mukoadhäsiven Eigenschaften von Polymeren jedoch oft nicht ausreichend, um den gewünschten Effekt zu erzielen. Der vorliegenden Erfindung liegt daher die Aufgabe zugrunde, die vorstehend aufgeführten Probleme des Stands der Technik zu überwinden und mukoadhäsive Polymere bereitzustellen, die hervorragende Eigenschaften besitzen.

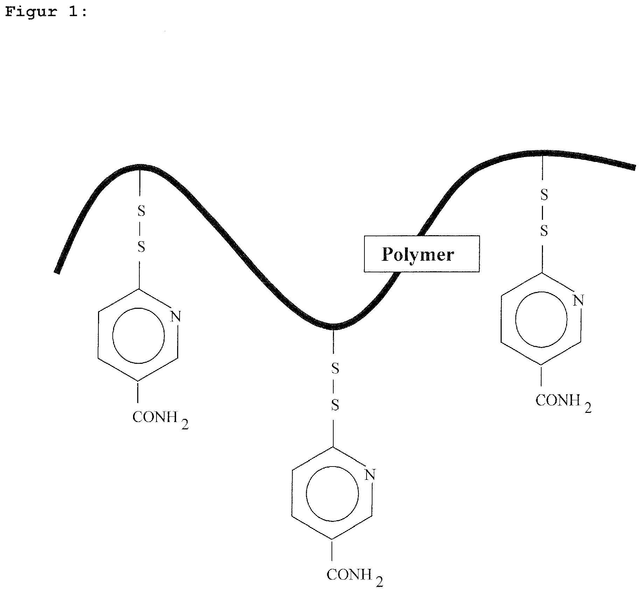

Diese Aufgabe wird erfindungsgemäß durch die polymeren Verbindungen gemäß Ansprüchen 1 und 3 bis 9 und deren Verwendung gemäß Ansprüchen 2, 10 und 11 gelöst. Erfindungsgemäß erfolgt eine Kopplung von Vitamin B-Derivaten wie Mercapto(iso)nikotinamiden oder Mercaptopyridoxinen an die freien Thiolgruppen von Thiomeren in Form von Disulfidbrücken, wie in Figur 1 anhand des 6-Mercaptonikotinamides gezeigt ist. Durch die Kopplung dieser Teilstrukturen an Thiomere reagieren diese deutlich schneller und zu einem vergleichsweise höheren Prozentsatz mit Thiolgruppen von Mukusglykoproteinen bzw. mit freien Thiolgruppen des Thiomers selbst. Neben Mercapto(iso)nikotinamiden und Mercaptopyridoxinen eignen sich zu diesem Zweck auch Mercaptonikotinsäuren, die zwar prinzipiell schon aus dem Stand der Technik bekannt sind (zB aus der CS 200 754), deren Verwendung in mukoadhäsiven Produkten aber noch nicht beschrieben wurde. Da diese jedoch zu zahlreichen Nebenwirkungen führen wie Vasodilatation oder Veränderungen im Fettstoffwechsel, sind Mercaptonikotinamide und Mercaptopyridoxine - also Vitamin B3- und Vitamin B6 Derivate - erfindungsgemäß bevorzugt. Als erfindungsgemäße Ausgangspolymere eignen sich vor allem thiolisierte quervernetzte) Poly(meth)acrylate, wie z.B. Polyacrylsäure-Cysteamin Konjugate [Hornbach et al., J Pharm Sci. 98 (2009) 555-564] oder Polycarbophil-Cystein Konjugate [Vetter et al., J Pharm Sci. 99 (2010) 1427-1439], thiolisierte Chitosane wie z.B. Chitosan-Thioglykolsäure Konjugate [Bernkop-Schnürch and Hopf Sci. Pharm., 69 (2001) 109-118], Chitosan-Mercaptonikotinsäure Konjugate [Millotti et al., Biomacromolecules, 10 (2009) 3023-3027] oder Chitosan-Glutathion-Konjugate [Kafedjiiski et al., Pharm Res., 22 (2005) 1480-1488], thiolisierte Pektine wie z.B. Pektin-Cystein Konjugate [Majzoob et al., J. Pharm. Pharmacol., 58 (2006) 1601-1610] oder Pectin-4-Mercaptoaminophenol Konjugat [Perera et al., AAPS PharmSciTech. , 11 (2010) 174-180], thiolisierte Alginate wie z.B. Alginat-Cystein Konjugate [Bernkop-Schnürch et al., J. Control. Release, 71 (2001) 277-285], thiolsierte Hyaluronsäure wie z.B. Hyaluronsäure-Cystein Ethylester Konjugate [Kafedjiiski et al., Int. J. Pharm., 343, (2007) 48-58], thiolisierte Polyallylamine wie z.B. Polyallylamin-Thioglykolsäure Konjugate, thiolisiertes Polylysin, thiolisiertes Polyornithin, thiolisierte Polyaminoamide, thiolisierte Zellulosederivate, wie z.B. Carboxymethylzellulose-Cystein Konjugate [Bernkop-Schnürch, Int. J. Pharm., 194, (2000) 239-247], thiolisierte (quervernetzte) Polyvinylpyrrolidone, die durch Zugabe von S-geschützten Thiolgruppen-tragenden Vinylverbindungen wie z.B. S-Acetyl-Cystein-Acrylamid während der Polymerisation von Vinylpyrrolidon entstehen, sowie thiolisierte (quervernetzte) (Meth)acrylsäure/Ethylacrylat Co-Polymere, die in Analogie zu thiolisierten Polyvinylpyrrolidonen hergestellt werden können. Zudem können neben den Monomeren Allylamin, Vinylpyrrolidon, (Meth)acrylsäure und Ethylacrylat auch weitere Monomere mit Vinylteilstruktur wie z.B. Vinylalkohol, Vinylimidazol, Vinylcaprolaktam oder (Meth)acrylamide mit S-geschützten Thiolgruppen-tragenden Vinylverbindungen im beliebigen Verhältnis der verschiedenen Monomere zueinander polymerisiert werden. Als weitere Thiolgruppentragende Liganden eignen sich zu dem Mercaptobenzoesäure, N-Acetyl-Cystein, Homocystein, 3-Thioproprionsäure, 4-Thiobutansäure, Thiobutylamidin sowie Thioethylamidin [Kafedjiiski et al., Biomaterials, 27 (2006) 127-135]. An diese mukoadhäsiven Polymere werden Mercaptonikotinamide bzw. Mercaptopyridoxine unter Ausbildung von Disulfidbrücken zwischen dem jeweiligen Thiomer und der freien Thiolgruppe des jeweiligen Vitamin B Derivates mittels Oxidation kovalent gebunden. Dabei ist gemäß einer bevorzugten Ausführungsform der Erfindung ein molarer Überschuss an Mercaptonikotinamid bzw. Mercaptopyridoxin gegenüber den freien Thiolgruppen des Polymers vorteilhaft. Wie bei Kamari et al. beschrieben, kann dieser Oxidationsprozess durch die Zugabe von Harnstoffperoxid und Maleinsäureanhydrid zusätzlich beschleunigt werden [Karami et al., Molecules, 10 (2005) 1385-63]. Desweitern kann in einer bevorzugten Ausführungsform die Oxidation auch durch Zugabe von Wasserstoffsuperoxid beschleunigt werden. Generell läuft der Oxidationsprozess bei höheren pH-Werten schneller ab. Die erfindungsgemäßen Polymere weisen 10-1000 µmol Mercapto(iso)nikotinamid bzw. Mercaptopyridoxin Teilstrukturen, und bevorzugt 100 bis 1000 µmol Mercapto(iso)nikotinamid bzw. Mercaptopyridoxin Teilstrukturen pro Gramm Polymer auf. Durch die zugegebene Menge an dem jeweiligen Vitamin B-Derivat kann die Anzahl an verbleibenden Thiolgruppen, die keine Disulfidbrücken mit dem Liganden ausbilden, kontrolliert werden.

Als erfindungsgemäß verwendbare Mercaptonikotinamide eignen sich vor allem 2-Mercaptonikotinamid und 6-Mercaptonikotinamid sowie 2- und 6-Mercaptoisonikotinamid. Die Herstellung von 6-Mercaptonikotinamid ist in Beispiel 1 beschrieben. Die Herstellung der oben genannten weiteren Derivate erfolgt in Analogie. Als erfindungsgemäßes Mercaptopyridoxin eignet sich vor allem 6-Mercaptopyridoxin. Zur Herstellung desselben wird von 6-Chloropyridoxin ausgegangen, dessen Synthese von Blackwood et al. [Blackwood et al., J. Am. Chem. Soc., 80 (1958) 6244-6249] beschrieben wird. Der Austausch des Chlors durch eine Mercaptogruppe erfolgt gemäß der in Beispiel 1 beschriebenen Methode.

Alternativ zu dieser Herstellungsmethode können gemäß einer bevorzugten Ausführungsform auch S-(2 bzw. 6-Mercapto(iso)nikotinamid)- bzw. S-(6-Mercaptopyridoxin)-Disulfide mit Vinylgruppe(n) tragenden Verbindungen wie z.B. Cystein-Acrylamid mit beliebigen anderen Vinylgruppe(n) tragenden Monomeren wie beispielsweise (Meth)acrylsäure, Vinylpyrrolidon, Vinylalkohol oder Ethylacrylat polymerisiert werden.

Eine weitere Möglichkeit zur Herstellung der erfindungsgemäßen Polymere liegt in der kovalenten Bindung von S-(2 bzw. 6-Mercapto(iso)nikotinamid)- bzw. S-(6-Mercaptopyridoxin)-Disulfiden mit voraktivierten Liganden an Polymere. So kann beispielsweise N-Succinimidyl 3-(2-Nikotinamidyldithio)-Propionat sehr effizient an Polymere mit primären Aminogruppen (z.B. Polyallylamin, Chitosan) unter Ausbildung von Amidbindungen gekoppelt werden.

Ein weiterer Vorteil dieser so hergestellten Verbindungen liegt darin, dass durch die Überführung von freien Thiolgruppen in Disulfide mit Mercaptonikotinamiden bzw. Mercaptopyridoxinen diese nicht mehr oxidationsempfindlich sind, da die Thiolgruppen bereits in der oxidierten Form vorliegen. Werden erfindungsgemäße Polymere mit Polymeren, die freie Thiolgruppen aufweisen, kombiniert, so kommt es zur Ausbildung von Disulfidbrücken zwischen diesen beiden Polymeren und damit zu einer vergleichsweise schnellen Viskositätszunahme. Dieser Effekt kann auch erzielt werden, wenn am Thiomer nicht alle Thiolgruppen in Disulfide mit Mercaptonikotinamiden bzw. Mercaptopyridoxinen übergeführt werden.

Aufgrund dieser mukoadhäsiven Eigenschaften bzw. der erhöhten Stabilität eignen sich die erfindungsgemäßen Polymere für eine breite Anwendung vor allem im pharmazeutischen und kosmetischen Bereich sowie in Health-Care-Produkten. Die Erfindung liefert daher auch die Verwendung der erfindungsgemäßen mukoadhäsiven Polymere als pharmazeutische und kosmetische Produkte, sowie als Health-Care-Produkte. Darüber hinaus wird erfindungsgemäß eine Zusammensetzung bereitgestellt, die eine oder mehrere erfindungsgemäße(s) Polymer(e), sowie Wirkstoffe), Hilfsstoff(e), und/oder Lösungsmittel umfaßt.

Neben diesen erhöhten mukoadhäsiven Eigenschaften werden durch (Iso)nikotinamid- bzw. Pyridoxin-Disulfid-Seitenketten erfindungsgemäß auch die permeationsbeschleunigenden Eigenschaften von Thiomeren deutlich erhöht. So konnte beispielsweise der Permeationskoeffizient (Papp) von einem Fluoreszenz markierten Dextran (Molekülmasse: 4,4 kDa; FD4) an der frisch entnommenen Dünndarmmukosa von der Ratte, der ohne Polymer Zugabe nach der von Föger et al. beschriebenen Methode [Föger et al., Amino Acid, 35 (2008) 233-241] mit 9,6 ± 3.5 x 107 cm/s bestimmt wurde, durch Zugabe von 0,5% (m/v) Chitosan-Thioglykolsäure Konjugat (Molekülmasse 450 kDa; 234 pmol Thiol-Gruppen pro Gramm Polymer) um das 1,8-fache jedoch durch Zugabe von 0,5% (m/v) desselben Polymers, bei dem zuvor sämtliche freien Thiolgruppen in Disulfide mit 6-Mercaptonikotinamid übergeführt wurden, um das 5,3-fache erhöht werden.

Die erfindungsgemäßen Polymere eignen sich im pharmazeutischen Bereich als Wirkstoffträgermatrix in Tabletten, als Gelbildner in halbfesten und flüssigen Zubereitungen, als adhäsive Wundauflagen, als Gerüststruktur im Bereich des Tissue Engineerings sowie zur Herstellung von mikro- und nanopartikulären Wirkstoffabgabesystemen. Im kosmetischen Bereich stehen vor allem Anwendungen als Haarbehandlungsmittel wie z.B. Haarstyling Gele, Fixierer, Färbemittel, Waschmittel und Umhüllungsmittel für Haar, Wimpern und Augenbraun im Vordergrund, da aufgrund der Proteinstruktur des Haars, der Nägel als auch der Haut Thiol-Teilstrukturen vorliegen, mit denen die erfindungsgemäßen Polymere reagieren können. Anwendungen in Nagellacken, Make-Ups und Antitranspirantien sind ebenfalls vorteilhaft. Da auch im Leder freie Thiolgruppen aufgrund von Cystein-Teilstrukturen in Proteinen vorliegen, eignen sich die erfindungsgemäßen Polymere auch zur LederImprägnierung. Eine Anwendung in Lacken sowie in verschiedenen Reinigungs- und Schmiermitteln ist mit den erfindungsgemäßen Polymeren ebenfalls möglich.

Die Erfindung umfaßt die folgenden Ausführungsformen:
(1) Polymere Verbindungen mit Thiolteilstrukturen, die in Form von Disulfiden mit 2- bzw. 6-Mercapto(iso)nikotinamid vorliegen.
(2) Polymere Verbindungen gemäß (1), dadurch gekennzeichnet, daß es sich um mukoadhäsive Polymere handelt, die im Mukoadhäsionstest am rotierenden Zylinder in Form von Testtabletten von 30 mg mit einem Durchmesser von 5 mm mehr als 8 Stunden und im Speziellen mehr als 24 Stunden an der Dünndarmmukosa vom Schwein anhaften.
(3) Polymere Verbindungen gemäß (1) oder (2), dadurch gekennzeichnet, daß es sich bei den polymeren Verbindungen um (quervernetzte) Poly(meth)acrylsäure, (trimethylierte) Chitosane, Hyaluronsäure, Pektine, Alginate, Methylzellulose, Hydroxyethylzellulose oder Natriumcarboxymethylzellulose handelt.
(4) Polymere Verbindungen gemäß (1) bis (3), dadurch gekennzeichnet, daß diese in Kombination mit Verbindungen, die mehr als eine Thiolgruppe aufweisen, zu einer Viskositätszunahme führen.
(5) Polymere Verbindungen gemäß (1) bis (4), dadurch gekennzeichnet, daß die Thiolisierung der in Anspruch 3 genannten Polymere durch die Ausbildung von Amidbindungen mit Cystein, Cysteamin, N-Acetylcystein, Thioglykolsäure, Mercaptobenzoesäure, Mercaptonikotinsäure, Glutathion oder Mercaptoanilin erfolgt.
(6) Polymere Verbindungen gemäß (1) bis (5), dadurch gekennzeichnet, daß 10-1000 µmol Mercapto(iso)nikotinamid Teilstrukturen und im Speziellen 100 bis 1000 µmol Mercapto(iso)nikotinamid-Teilstrukturen pro Gramm Polymer vorliegen.
(7) Polymere Verbindungen gemäß (1) bis (6), dadurch gekennzeichnet, daß diese als Hilfsstoffe in pharmazeutischen, kosmetischen bzw. in Health-Care-Produkten verwendet werden.
(7) Polymere Verbindungen gemäß (1) bis (6), dadurch gekennzeichnet, daß diese zur Lederimprägnierung verwendet werden.

### Beispiele:

Die folgenden Beispiele sind exemplarisch und sollen die hier offenbarte Erfindung illustrieren. Änderungen und Variationen der folgenden Beispiele im Rahmen der vorliegenden Patentansprüche können durchgeführt werden.

### Beispiel 1

### Synthese von 6-Mercaptonikotinamid

5,0 g 6-Chlornikotinamid (31,9 mmol) sowie 2,65 g Thioharnstoff (34,8 mmol) werden in absolutem Ethanol (50 ml) suspendiert und 6 Stunden unter Rückfluss erhitzt (Badtemperatur ca. 90°C), wobei sich die Mischung mit der Zeit gelb färbt. Anschließend wird das Reaktionsgemisch auf Raumtemperatur abgekühlt. Das entstandene *S*-(5-Carbamyl-2-pyridyl)thiuroniumchlorid wird abfiltriert und getrocknet. Es werden 6,7 g Rohprodukt (90 %) als gelbes Pulver isoliert. ¹H-NMR (200 MHz, DMSO-d₆, δ): 7,76 (br s, 1 H) ; 7,83 (d, 1 H, J = 8,4 Hz); 8,33 (dd, 1 H, J = 8,4 Hz, J = 2,0 Hz); 8,38 (br s, 1 H) ; 9,05 (d, 1 H, J = 2,0 Hz); 9,69 (br s, 4 H). Die 6,7 g *S-*(5-Carbamyl-2-pyridyl)thiuroniumchlorid (28,8 mmol) werden in Wasser (30 ml) suspendiert und mit 5 M NaOH (20 ml) versetzt. Die Suspension wird 30 Minuten bei Raumtemperatur gerührt und anschließend vorsichtig mit Eisessig angesäuert (pH 4,9). Das Rohprodukt wird abfiltriert und aus einem geeigneten Lösungsmittel (z.B. Wasser) umkristallisiert. Es werden 3,4 g Produkt (69 % bezogen auf 6-Chlornikotinamid) als gelbes Pulver gewonnen. ¹H-NMR (200 MHz, DMSO-d₅, δ): 7,29 (d, 1 H, J = 9,1 Hz); 7,46 (br s, 1 H); 7,76 (dd, 1 H, J = 9,1 Hz, J = 2,2 Hz); 7,95 (br s, 1 H) ; 8,13 (d, 1 H, J = 2,2 Hz) ; 13,74 (br s, 1 H).

### Beispiel 2

### Synthese von Polyacrylsäure-Cystein-6-Mercaptonikotinamid Konjugaten

Ein Gramm Polyacrylsäure 450 kDa (Sigma-Aldrich, Wien) wird in 200 ml demineralisiertem Wasser hydratisiert und der pH-Wert durch Zugabe von 1 M NaOH auf pH 5 eingestellt. Die Carbonsäuregruppen des Polymers werden durch Zugabe von 1-Ethyl-3-(3-dimethylaminopropyl) Carbodiimid Hydrochlorid in einer Endkonzentration von 200 mM bei Raumtemperatur unter Rühren 30 Minuten voraktiviert. Nach der Zugabe von 1 g Cystein wird der pH-Wert der Lösung gegebenenfalls auf pH 5 mit HCl bzw. NaOH nachjustiert und der Reaktionsansatz sechs Stunden bei Raumtemperatur gerührt. Das entstandene Polyacrylsäure-Cystein Konjugat wird gegen eine wässrige 1 mM HCl Lösung, zweimal gegen das gleiche Dialysemedium jedoch zusätzlich 1% NaCl enthaltend und abschließend erschöpfend gegen Wasser bei 10°C unter Lichtausschluss dialysiert. Im Anschluss daran wird der pH-Wert des Konjugates mit 1 M NaOH auf pH 5 eingestellt. Das isolierte Konjugat wird bei -30°C gefriergetrocknet. Die Aufbewahrung erfolgt bei 4°C. Die Konzentration an kovalent gebundenen Thiolgruppen wird mit Ellman's Reagens bestimmt.

Das so hergestellte Thiomer weist 50-250 µmol kovalent gebundene Thiolgruppen pro Gramm Polymer auf.

2,50 g eines so hergestellten Konjugates (65 pmol SH/g Polymer) werden in Wasser (200 ml) gelöst. Die Lösung wird mit 1 M NaOH auf pH 6 alkalisiert und mit 50 mg 6-Mercapto-nicotinamid bzw 2-Mercaptonicotinamid (in 5 ml DMS0 und 5 ml Wasser gelöst) sowie 20 mg Harnstoffperoxid und 30 mg Maleinsäureanhydrid (gemeinsam in 10 ml Wasser gelöst) versetzt. Die Lösung wird 24 Stunden bei Raumtemperatur gerührt, Anschließend wird erschöpfend gegen demineralisiertes Wasser dialysiert und in der Folge lyophilisiert.

### Beispiel 3

### Synthese von Chitosan-Thioglykolsäure-2- bzw. 6-Mercaptonikotinamid-Konjugaten

Ein Gramm Chitosan werden in 10 ml 1 M HCl hydratisiert und anschließend mit demineralisiertem Wasser auf eine Endkonzentration von 1% (m/v) verdünnt. Es werden 1 g Thioglykolsäure sowie 1-Ethyl-3-(3-dimethylaminopropyl) Carbodiimid Hydrochlorid in einer Endkonzentration von 200 mM zugegeben und der pH gegebenenfalls auf pH 5 mit 1 M HCl bzw. 1 M NaOH nachjustiert. Der Reaktionsansatz wird sechs Stunden bei Raumtemperatur gerührt. Das Konjugat wird gegen eine wässrige 1 mM HCl Lösung, zweimal gegen das gleiche Dialysemedium jedoch zusätzlich 1% NaCl enthaltend und abschließend erschöpfend gegen Wasser bei 10°C unter Lichtausschluss dialysiert. Im Anschluss daran wird der pH-Wert des Konjugates mit 1 M NaOH auf pH 5 eingestellt. Das isolierte Konjugat wird bei -30°C gefriergetrocknet. Die Aufbewahrung erfolgt bei 4°C. Die Konzentration an kovalent gebundenen Thiolgruppen wird mit Ellman's Reagens bestimmt.

2,50 g Chitosan-TGA (550 µmol SH/g Polymer) werden in Wasser (200 ml) gelöst. Die Lösung wird mit 1 M NaOH auf pH 6 eingestellt und mit 215 mg 6-Mercaptonicotinamid bzw 2-Mercaptonicotinamid (in 5 ml DMSO + 5 ml Wasser gelöst) sowie 130 mg Harnstoffperoxid (in 10 ml Wasser gelöst) versetzt. Die Lösung wird 24 Stunden bei Raumtemperatur gerührt. Anschließend wird erschöpfend gegen demineralisiertes Wasser dialysiert und in der Folge lyophilisiert.

### Beispiel 4

### Synthese von Pectin-4-Aminothiophenol-2-Mercaptonikotinamid-Konjugaten

Ein Gramm Pektin werden in 250 ml Wasser/Dioxan (2+1) gelöst. Nach kontinuierlicher Zugabe von 0,2 g 4-Mercaptoanilin gelöst in 3 ml Dioxan wird der pH-Wert mit 0,5 M NaOH auf 4.5 eingestellt und 1-Ethyl-3-(3-dimethylaminopropyl) Carbodiimid Hydrochlorid in einer Endkonzentration von 200 mM zugegeben. Nach drei Stunden Rühren bei Raumtemperatur wird der pH-Wert auf 7,5 eingestellt und es werden 0,2 g Natriumborhydrid zugegeben. Der Reaktionsansatz wird eine Stunde bei zu gerührt. In der Folge wir nicht gebundenes 4-Mercaptoanilin durch mehrmaliges Ausschütteln des Reaktionsansatzes mit Ethylacetat entfernt. Das entstandene Konjugat wird durch Zugabe von Isopropanol gefällt und der Niederschlag wird mit reinem Isopropanol und Aceton gewaschen. Das gereinigte Konjugat wird im Exsiccator getrocknet. 0,5 Gramm Pectin-4-Aminothiophenol Konjugat (420 µmol SH/g Polymer) werden in Wasser (200 ml) gequollen. Die Lösung wird mit 1 M NaOH auf pH 6,5 eingestellt und mit 500 mg 2-Mercaptonicotinamid (in 25 ml DMSO + 25 ml Wasser) versetzt. Die Lösung wird 24 Stunden bei Raumtemperatur gerührt. Anschließend wird erschöpfend gegen demineralisiertes Wasser dialysiert und in der Folge lyophilisiert.

### BEISPIEL 5

### Mukoadhäsionstest

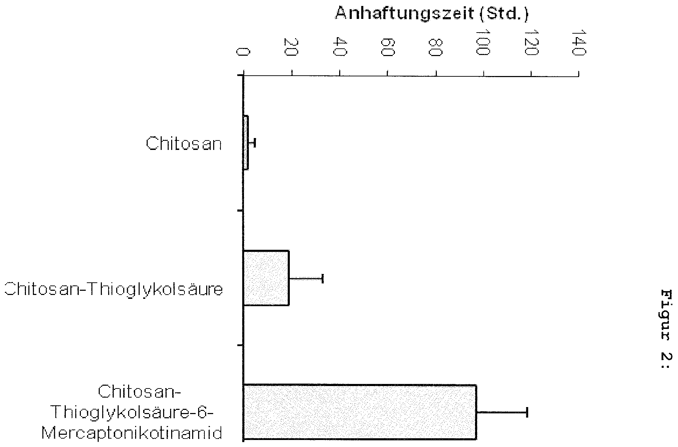

Die mukoadhäsiven Eigenschaften, der gemäß Beispiel 3 hergestellten Polymere, werden nach der von Bernkop-Schnürch et al. beschriebenen Methode [Bernkop-Schnürch et al., Int. J. Pharm., 260 (2003) 229-237] bestimmt. Dazu werden die Polymere mit und ohne Mercaptonikotinamid Teilstrukturen zu 30 mg Tabletten mit einem Durchmesser von 5 mm verpresst. Im Anschluss werden diese auf eine frisch entnommene Dünndarmmukosa vom Schwein, die auf einen Stahlzylinder aufgespannt ist, mit leichtem Druck angebracht. Der Zylinder wird in einem Dissolutionstester gemäß dem Europäischen Arzneibuch in 50 mM Phosphatpuffer pH 6.5 von 37°C in eine Rotation von 100 Umdrehungen pro Minute versetzt. Der Zeitpunkt des Ablösens der Tabletten von der Mukosa wird visuell bestimmt. Die Ergebnisse dieser Studie sind in Figur 2 graphisch dargestellt (Mittelwert ± Standardabweichung; n=4).

### BEISPIEL 6

### Rheologische Studien

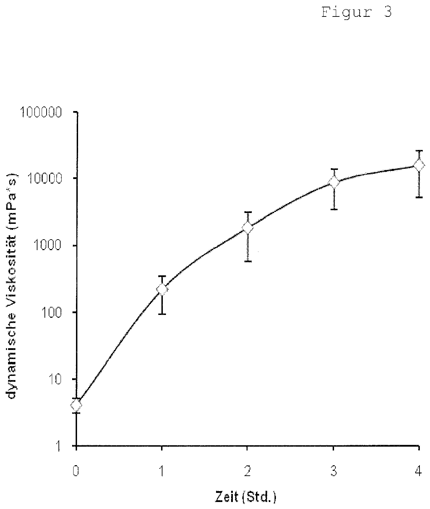

Zunächst werden 0,25 g von Chitosan-Thioglykolsäure-6-Mercaptonikotinamid in 50 ml demineralisiertem Wasser hydratisiert. Das entstehende Gel wird zu 50 ml einer 0,5%igen (m/v) Chitosan-Thioglykolsäure Lösung gegeben. Der Reaktionsansatz wird homogenisiert und auf pH 6.0 eingestellt. Zu in Figur 3 ersichtlichen Zeitpunkten wird die Viskosität des Gels gemessen (oszillierende Messung bei konstanter Frequenz von 1 Hz). Es kommt wie in Figur 3 gezeigt innerhalb von weniger Stunden zu einer Zunahme der Viskosität um das mehr als 1000-Fache (Mittelwert ± Standardabweichung; n=4).

### Beispiel 7

### Herstellung von Tabletten

20 g von Polyacrylsäure-Cystein-6-Mercaptonikotinamid (Beispiel 2) werden mit 1 g Miconazol homogenisiert und zu 0,2 g schweren Tabletten direkt verpresst. Die Tabletten zeigen einen ausreichend hohen Zusammenhalt sowie eine kontrollierte Freisetzung des Antimykotikums.

### Beispiel 8

### Herstellung von Nasentropfen

0,1 g von Chitosan-Thioglykolsäure-6-Mercaptonikotinamid (Beispiel 3) und 0,05 g Oxymetazolin HCl werden in 100 ml demineralisiertem Wasser gelöst und in 10 ml Behältnisse mit Tropfaufsatz abgefüllt. Optional werden Benzalkoniumchlorid in einer Endkonzentration von 0,015% (m/v) und Edetinsäuredinatriumdihydrogenat 0,05% (m/v) als Konservierungsmittel zugesetzt.

### Beispiel 9

### Herstellung eines Haargels

0,25 g von Chitosan-Thioglykolsäure-6-Mercaptonikotinamid (Beispiel 3) und 0,25 g von Chitosan-Thioglykolsäure (Beispiel 3)werden in 100 ml Wasser/Isopropanol (9+1) gequollen. Der pH-Wert wird gegebenenfalls mit 1 M HCl bzw. 1 M NaOH auf 6,0 nachjustiert. Das entstandene Gel wird zu je 10 ml in Sachets aus Aluminium/Kunststoff Verbundmaterial abgefüllt. Nach dem Öffnen der Sachets und dem Verteilen auf dem Haar kommt es zu einer starken Viskositätszunahme des Gels und damit zu einer Struktur- und Halt-gebenden Wirkung.

## Patentansprüche

1. Polymere Verbindung, umfassend 2-Nikotinamid-Disulfid Seitenketten, 2-Isonikotinamid-Disulfid Seitenketten, 6-Nikotinamid-Disulfid Seitenketten, 6-Isonikotinamid-Disulfid Seitenketten oder 6-Pyridoxin-Disulfid-Seitenketten.

2. Polymere Verbindung nach Anspruch 1 zur Verwendung in mucoadhäsiven Produkten.

3. Polymere Verbindung nach Anspruch 1, umfassend 2-Nikotinamid-Disulfid Seitenketten, 2-Isonikotinamid-Disulfid Seitenketten, 6-Nikotinamid-Disulfid Seitenketten oder 6-Isonikotinamid-Disulfid Seitenketten.

4. Polymere Verbindung nach Anspruch 1, wobei es sich um
S-(2- bzw. 6-Mercapto(iso)nikotinamid)- bzw. S-(6-Mercaptopyridoxin)-Cystein-Disulfid-Seitenketten,
S-(2- bzw. 6-Mercapto(iso)nikotinamid)- bzw. S-(6-Mercaptopyridoxin)-Homocystein-Disulfid-Seitenketten,
S-(2- bzw. 6-Mercapto(iso)nikotinamid)- bzw. S-(6-Mercaptopyridoxin)-Cysteamin-Disulfid-Seitenketten,
S-(2- bzw. 6-Mercapto(iso)nikotinamid)- bzw. S-(6-Mercaptopyridoxin)-N-Acetylcystein-Disulfid-Seitenketten,
S-(2- bzw. 6-Mercapto(iso)nikotinamid)- bzw. S-(6-Mercaptopyridoxin)-Thioglycolsäure-Disulfid-Seitenketten,
S-(2- bzw. 6-Mercapto(iso)nikotinamid)- bzw. S-(6-Mercaptopyridoxin)-3-Thiopropionsäure-Disulfid-Seitenketten,
S-(2- bzw. 6-Mercapto(iso)nikotinamid)- bzw. S-(6-Mercaptopyridoxin)-4-Thiobutansäure-Disulfid-Seitenketten,
S-(2- bzw. 6-Mercapto(iso)nikotinamid)- bzw. S-(6-Mercaptopyridoxin)-Mercaptobenzoesäure-Disulfid-Seitenketten,
S-(2- bzw. 6-Mercapto(iso)nikotinamid)- bzw. S-(6-Mercaptopyridoxin)-Mercaptonikotinsäure-Disulfid-Seitenketten,
S-(2- bzw. 6-Mercapto(iso)nikotinamid)- bzw. S-(6-Mercaptopyridoxin)-Glutathion-Disulfid-Seitenketten,
S-(2- bzw. 6-Mercapto(iso)nikotinamid)- bzw. S-(6-Mercaptopyridoxin)-Thioethylamidin-Disulfid-Seitenketten,
S-(2- bzw. 6-Mercapto(iso)nikotinamid)- bzw. S-(6-Mercaptopyridoxin)-4-Thiobutylamidin-Disulfid-Seitenketten oder
S-(2- bzw. 6-Mercapto(iso)nikotinamid)- bzw. S-(6-Mercaptopyridoxin)-Mercaptoanilin-Disulfid-Seitenketten handelt, die über eine Amid-, Amidin- oder Esterbindung an das Polymer gebunden sind,
wobei S-(2- bzw. 6-Mercapto(iso)nicktinamid)- für S-(2-Mercaptonikotinamid)-, S-(2-Mercaptoisonikotinamid)-, S-(6-Mercaptonikotinamid)- bzw. S-(6-Mercaptoisonikotinamid)- steht.

5. Polymere Verbindung nach Anspruch 1, worin das Polymer ausgewählt ist aus
- thiolisierten (quervernetzten) Poly(meth)acrylaten,
- thiolisierten Chitosanen,
- thiolisierten Pektinen,
- thiolisierten Alginaten,
- thiolisierter Hyaluronsäure,
- thiolisierten Polyallylaminen,
- thiolisiertem Polylysin,
- thiolisiertem Polyornithin,
- thiolisierten Polyaminoamiden,
- thiolisierten Zellulosederivativen,
- thiolisierten (quervernetzen) Polyvinylpyrrolidonen, die durch Zugabe von S-geschützten,Thiolgruppen-tragenden Vinylverbindungen während der Polymerisation entstehen, und
- thiolisierten (quervernetzten) (Meth)acrylsäure /Ethylacrylat Copolymeren.

6. Polymere Verbindung nach Anspruch 1, wobei 10 bis 1000 µmol Mercaptonikotinamid-, Mercaptoisonikotinamid- oder Mercaptopyridoxin-Teilstrukturen pro Gramm Polymer vorliegen.

7. Polymere Verbindung nach Anspruch 1, wobei 100 bis 1000 µmol Mercaptonikotinamid-, Mercaptoisonikotinamid- oder Mercaptopyridoxin-Teilstrukturen pro Gramm Polymer vorliegen.

8. Polymere Verbindung nach Anspruch 1, worin das Polymer ausgewählt ist aus
- thiolisierten (quervernetzten) Poly(meth)acrylaten,
- thiolisierten Chitosanen,
- thiolisierten Pektinen,
- thiolisierten Alginaten,
- thiolisierter Hyaluronsäure,
- thiolisierten Polyallylaminen,
- thiolisiertem Polylysin,
- thiolisiertem Polyornithin,
- thiolisierten Polyaminoamiden,
- thiolisierten Zellulosederivativen,
- thiolisierten (quervernetzen) Polyvinylpyrrolidonen, die durch Zugabe von S-geschützten,Thiolgruppen-tragenden Vinylverbindungen während der Polymerisation entstehen, und
- thiolisierten (quervernetzten) (Meth)acrylsäure /Ethylacrylat Copolymeren, umfassend
- S-(2- bzw. 6-Isonikotinamid)-Disulfid-Seitenketten
- S-(6-Pyridoxin)-Disulfid-Seitenketten

9. Polymere Verbindung nach einem der Ansprüche 5 oder 8, worin die polymere Verbindung ausgewählt ist aus thiolisiertem
- Polyacrylat,
- Chitosan,
- Pectin.

10. Polymere Verbindung, umfassend 2- oder 6-Nikotinamid-Disulfid Seitenketten, 2- oder 6-Isonikotinamid-Disulfid Seitenketten, Nikotinsäure-Disulfid-Seitenketten oder 6-Pyridoxin-Disulfid-Seitenketten, zur therapeutischen Verwendung.

11. Verwendung einer polymeren Verbindung, umfassend 2- oder 6-Nikotinamid-Disulfid-Seitenketten, 2- oder 6-Isonikotinamid-Disulfid-Seitenketten, Nikotinsäure-Disulfid-Seitenketten oder 6-Pyridoxin-Disulfid-Seitenketten, für die Herstellung eines kosmetischen oder Health-Care-Produktes.

## Claims

1. A polymeric compound, comprising 2-nicotinamide-disulfide side chains, 2-isonicotinamide-disulfide side chains, 6-nicotinamide-disulfide side chains, 6-isonicotinamide-disulfide side chains or 6-pyridoxine-disulfide side chains.

2. A polymeric compound according to claim 1 for the use in muco-adhesive products.

3. A polymeric compound according to claim 1, comprising 2-nicotinamide-disulfide side chains, 2-isonicotinamide-disulfide side chains, 6-nicotinamide-disulfide side chains or 6-isonicotinamide-disulfide side chains.

4. A polymeric compound according to claim 1, wherein said side chains are
S-(2- or 6-mercapto(iso)nicotinamide)- or S-(6-mercaptopyridoxine)-cysteine-disulfide side chains, respectively,
S-(2- or 6-mercapto(iso)nicotinamide)- or S-(6-mercaptopyridoxine)-homocysteine-disulfide side chains, respectively,
S-(2- or 6-mercapto(iso)nicotinamide)- or S-(6-mercaptopyridoxine)-cysteamine-disulfide side chains, respectively,
S-(2- or 6-mercapto(iso)nicotinamide)- or S-(6-mercaptopyridoxine)-N-acetylcysteine-disulfide side chains, respectively,
S-(2- or 6-mercapto(iso)nicotinamide)- or S-(6-mercaptopyridoxine)-thioglycolic acid-disulfide side chains, respectively,
S-(2- or 6-mercapto(iso)nicotinamide)- or S-(6-mercaptopyridoxine)-3-thiopropionic acid-disulfide side chains, respectively,
S-(2- or 6-mercapto(iso)nicotinamide)- or S-(6-mercaptopyridoxine)-4-thiobutanoic acid-disulfide side chains, respectively,
S-(2- or 6-mercapto(iso)nicotinamide)- or S-(6-mercaptopyridoxine)-mercaptobenzoic acid-disulfide side chains, respectively,
S-(2- or 6-mercapto(iso)nicotinamide)- or S-(6-mercaptopyridoxine)-mercaptonicotinic acid-disulfide side chains, respectively,
S-(2- or 6-mercapto(iso)nicotinamide)- or S-(6-mercaptopyridoxine)-glutathione-disulfide side chains, respectively,
S-(2- or 6-mercapto(iso)nicotinamide)- or S-(6-mercaptopyridoxine)-thioethylamidine-disulfide side chains, respectively,
S-(2- or 6-mercapto(iso)nicotinamide)- or S-(6-mercaptopyridoxine)-4-thiobutylamidine-disulfide side chains, respectively, or
S-(2- or 6-mercapto(iso)nicotinamide)- or S-(6-mercaptopyridoxine)-mercaptoaniline-disulfide side chains, respectively, being attached to the polymer via amide, amidine or ester bonds,
wherein S-(2- or 6-mercapto(iso)nicotinamide)- represents S-(2-mercaptonicotinamide)-, S-(2-mercaptoisonicotinamide)-, S-(6-mercaptonicotinamide)- or S-(6-mercaptoisonicotinamide)-.

5. A polymeric compound according to claim 1, wherein the polymer is selected from
- thiolated (cross-linked) poly(meth) acrylates,
- thiolated chitosans,
- thiolated pectins,
- thiolated alginates,
- thiolated hyaluronic acid,
- thiolated polyallyl amines,
- thiolated polylysine,
- thiolated polyornithine,
- thiolated polyamino amides,
- thiolated cellulose derivatives,
- thiolated (cross-linked) polyvinyl pyrrolidones, which are formed by the addition of S-protected, thiol moieties carrying vinyl compounds during the polymerisation, and
- thiolated (cross-linked) (meth)acrylic acid / ethyl acrylate co-polymers.

6. A polymeric compound according to claim 1, wherein 10 to 1000 µmol mercaptonicotinamide, mercaptoisonicotinamide or mercaptopyridoxine partial structures are present per gram of polymer.

7. A polymeric compound according to claim 1, wherein 100 to 1000 µmol mercaptonicotinamide, mercaptoisonicotinamide or mercaptopyridoxine partial structures are present per gram of polymer.

8. A polymeric compound according to claim 1, wherein the polymer is selected from
- thiolated (cross-linked) poly(meth) acrylates,
- thiolated chitosans,
- thiolated pectins,
- thiolated alginates,
- thiolated hyaluronic acid,
- thiolated polyallyl amines,
- thiolated polylysine,
- thiolated polyornithine,
- thiolated polyamino amides,
- thiolated cellulose derivatives,
- thiolated (cross-linked) polyvinyl pyrrolidones, which are formed by the addition of S-protected, thiol moieties carrying vinyl compounds during the polymerisation, and
- thiolated (cross-linked) (meth)acrylic acid / ethyl acrylate co-polymers,
comprising
- S-(2- or 6-isonicotinamide)-disulfide side chains
- S-(6-pyridoxine)-disulfide side chains.

9. A polymeric compound according to any of claims 5 or 8, wherein the polymeric compound is selected from thiolated
- polyacrylate,
- chitosan,
- pectin.

10. A polymeric compound, comprising 2- or 6-nicotinamide-disulfide side chains, 2- or 6-isonicotinamide-disulfide side chains, nicotinic acid-disulfide side chains or 6-pyridoxine-disulfide side chains, for the therapeutic use.

11. Use of a polymeric compound, comprising 2- or 6-nicotinamide-disulfide side chains, 2-or 6-isonicotinamide-disulfide side chains, nicotinic acid-disulfide side chains or 6-pyridoxine-disulfide side chains, for the production of a cosmetic or health care product.

## Revendications

1. Composé polymère, comprenant des chaînes latérales de disulfure de 2-nicotinamide, des chaînes latérales de disulfure de 2-isonicotinamide, des chaînes latérales de disulfure de 6-nicotinamide, des chaînes latérales de disulfure de 6-isonicotinamide ou des chaînes latérales de disulfure de 6-pyridoxine.

2. Composé polymère selon la revendication 1 destiné à être utilisé dans des produits muco-adhésifs.

3. Composé polymère selon la revendication 1, comprenant des chaînes latérales de disulfure de 2-nicotinamide, des chaînes latérales de disulfure de 2-isonicotinamide, des chaînes latérales de disulfure de 6-nicotinamide ou des chaînes latérales de disulfure de 6-isonicotinamide.

4. Composé polymère selon la revendication 1, dans lequel il s'agit de
chaînes latérales de disulfure de S-(2- ou 6-mercapto(iso)nicotinamide) ou S-(6-mercaptopyridoxine)-cystéine,
chaînes latérales de disulfure de S-(2- ou 6-mercapto(iso)nicotinamide) ou S-(6-mercaptopyridoxine)-homocystéine,
chaînes latérales de disulfure de S-(2- ou 6-mercapto(iso)nicotinamide) ou S-(6-mercaptopyridoxine)-cystéamine,
chaînes latérales de disulfure de S-(2- ou 6-mercapto-(iso)nicotinamide) ou S-(6-mercaptopyridoxine)-N-acétylcystéine,
chaînes latérales de disulfure de l'acide S-(2- ou 6-mercapto(iso)nicotinamide)- ou S-(6-mercaptopyridoxine)-thioglycolique,
chaînes latérales de disulfure de l'acide S-(2- ou 6-mercapto(iso)nicotinamide)- ou S-(6-mercaptopyridoxine)-3-thiopropionique,
chaînes latérales de disulfure de l'acide S-(2- ou 6-mercapto(iso)nicotinamide)- ou S-(6-mercaptopyridoxine)-4-thiobutanoïque,
chaînes latérales de disulfure de l'acide S-(2- ou 6-mercapto(iso)nicotinamide)- ou
S-(6-mercaptopyridoxine)-mercaptobenzoïque,
chaînes latérales de disulfure de de l'acide S-(2- ou 6-mercapto(iso)nicotinamide)-ou S-(6-mercaptopyridoxine)-mercaptonicotinique,
chaînes latérales de disulfure de S-(2- ou 6-mercapto(iso)nicotinamide)- ou S-(6-mercaptopyridoxine)-glutathion,
chaînes latérales de disulfure de S-(2- ou 6-mercapto(iso)nicotinamide)- ou S-(6-mercaptopyridoxine)-thioéthylamidine,
chaînes latérales de disulfure de S-(2- ou 6-mercapto(iso)nicotinamide)- ou S-(6-mercaptopyridoxine)-4-thiobutylamidine, ou
chaînes latérales de disulfure de S-(2- ou 6-mercapto(iso)nicotinamide)- ou S-(6-mercaptopyridoxine)-mercaptoaniline, qui sont liées au polymère par une liaison amide, amidine ou ester,
dans lequel S-(2- ou 6-mercapto(iso)nicotinamide) représente S-(2-mercaptonicotinamide), S-(2-mercaptoisonicotinamide), S-(6-mercaptonicotinamide) ou S-(6-mercaptoisonicotinamide).

5. Composé polymère selon la revendication 1, dans lequel le polymère est sélectionné parmi
poly(méth)acrylates thiolisés (réticulés transversalement),
chitosanes thiolisés,
pectines thiolisées,
alginates thiolisés,
acide hyaluronique thiolisé,
polyallylamines thiolisées,
polylysine thiolisée,
polyornithine thiolisée,
polyaminoamides thiolisés,
dérivés cellulosiques thiolisés,
polyvinylpyrrolidones thiolisés (réticulés transversalement), qui sont obtenus par addition de composés vinyliques protégés S, portant des groupes thiol pendant la polymérisation, et
copolymères acide (méth)acrylique/acrylate d'éthyle thiolisés (réticulés transversalement).

6. Composé polymère selon la revendication 1, dans lequel il se trouve 10 à 1000 µmoles par gramme de polymère de structures partielles de mercaptonicotinamide, mercaptoisonicotinamide ou mercaptopyridoxine.

7. Composé polymère selon la revendication 1, dans lequel il se trouve 100 à 1000 µmoles par gramme de polymère de structures partielles de mercaptonicotinamide, mercaptoisonicotinamide ou mercaptopyridoxine.

8. Composé polymère selon la revendication 1, dans lequel le polymère est sélectionné parmi
poly(méth)acrylates thiolisés (réticulés transversalement),
chitosanes thiolisés,
pectines thiolisées,
alginates thiolisés,
acide hyaluronique thiolisé,
polyallylamines thiolisées,
polylysine thiolisée,
polyornithine thiolisée,
polyaminoamides thiolisés,
dérivés cellulosiques thiolisés,
polyvinylpyrrolidones thiolisées (réticulées transversalement), qui sont obtenus par addition de composés vinyliques protégés S, portant des groupes thiol pendant la polymérisation, et
copolymères acide (méth)acrylique/acrylate d'éthyle thiolisés (réticulés transversalement),
comprenant
- des chaînes latérales de disulfure de S-(2- ou 6-isonicotinamide)
- des chaînes latérales de disulfure de S-(6-pyridoxine).

9. Composé polymère selon une des revendications 5 ou 8, dans lequel le composé polymère est sélectionné parmi
- polyacrylate,
- chitosane,
- pectine,
thiolisé(e).

10. Composé polymère, comprenant des chaînes latérales de disulfure de 2- ou 6-nicotinamide, des chaînes latérales de disulfure de 2- ou 6-isonicotinamide, des chaînes latérales de disulfure de l'acide nicotinique ou des chaînes latérales de disulfure de 6-pyridoxine, pour une utilisation thérapeutique.

11. Utilisation d'un composé polymère, comprenant des chaînes latérales de disulfure de 2- ou 6-nicotinamide, des chaînes latérales de disulfure de 2- ou 6-isonicotinamide, des chaînes latérales de disulfure de l'acide nicotinique ou des chaînes latérales de disulfure de 6-pyridoxine, pour la production d'un produit cosmétique ou de soin.
